# EUROPÄISCHE PATENTANMELDUNG

(11) **EP 3 000 359 A1**
(43) Veröffentlichungstag der Anmeldung: **30.03.2016**
(21) Anmeldenummer: 15186954.2
(22) Anmeldetag: 25.09.2015
(51) Int. Cl.: A47C 31/12

(54) **SITZEINRICHTUNG**

(30) Priorität: 26.09.2014 CH 14592014
(71) Anmelder: Sitag AG, 9466 Sennwald (CH)
(72) Erfinder: Boduk, Yilmaz, 9466 Sennwald (CH)
(74) Vertreter: Riederer Hasler & Partner Patentanwälte AG

(57) **Zusammenfassung**

Die Erfindung betrifft eine Sitzeinrichtung, insbesondere Bürosessel, mit einem Sitzelement, einer Rückenlehne, einem Trägergestell an welchem das Sitzelement und die Rückenlehne bevorzugt mit einer Vielzahl von Gelenken angeordnet sind und an der Sitzeinrichtung angeordnete Sensoren. Die Sensoren erfassen von der Sitzeinrichtung erzeugte physikalische und/oder chemische Eigenschaften.

## Beschreibung

### Gebiet der Erfindung

Die Erfindung betrifft eine Sitzeinrichtung gemäss Oberbegriff des Anspruchs 1 und einen Nachrüstsatz für eine Sitzeinrichtung gemäss Anspruch 11.

### Stand der Technik

Aus dem Stand der Technik sind Bürostühle bekannt, deren Sitz- und/oder Lehnenflächen mit Drucksensor-Matten ausgerüstet sind.

In der WO 2005/074754 ist eine Drucksensor-Matte beschrieben, welche die Körperhaltung eines auf einem Bürostuhl Sitzenden verbessern soll. Ein Prozessor vergleicht die von der Matte erhaltenen Daten mit in einem Speicher hinterlegten Daten. Dadurch wird eine schlechte Körperhaltung des Sitzenden erkannt. Der Sitzende kann beispielsweise über einen Bildschirm auf eine schlechte Haltung hingewiesen werden. Die Drucksensoren reagieren jedoch nur auf das Körpergewicht des Sitzenden.

Die WO 03/001946 A1 offenbart einen Bürostuhl mit Sensoren, welche Sensoren an dem Bürostuhl überall dort angeordnet sind, wo der Bürostuhl mit einer auf dem Bürostuhl sitzenden Person in Berührung kommt. Die Sensoren erfassen ergonomische Daten der Person. Die erfassten Daten werden von einem Prozessor herangezogen, um ergonomische Probleme zu vermeiden, wenn die Person auf dem Bürostuhl sitzt. Wird eine ergonomisch ungünstige Sitzposition erkannt, so wird der Sitzende visuell oder akustisch gewarnt. Der Bürostuhl kann auch mit einer kabellosen Schnittstelle mit einem externen Computer verbunden sein, wodurch ein Sitzender mit dem Prozessor des Bürostuhls kommunizieren kann, beispielsweise seine Körper- und Anmeldedaten eingeben. Die Sensoren sind ausschliesslich dafür ausgelegt, das Vorhandensein einer Person, die Position des Körpers des auf dem Bürostuhl Sitzenden und dessen Bewegungen zu erfassen. Die Sensoren können Drucksensoren, Temperatursensoren, Lagesensoren oder eine Karners sein.

In der US 2011/0170945 A1 ist ein bewegbarer Kinosessel beschrieben, welcher auch einen Gewichtssensor und einen Prozessor umfasst. Basierend auf den Sensordaten wird der Grad der Bewegung des Sessels bestimmt. Der Sensor des Kinosessels ist lediglich dafür ausgelegt eine Eigenschaft des Kinobesuchers, beispielsweise dessen Gewicht, zu erfassen.

### Aufgabe der Erfindung

Aufgabe der vorliegenden Erfindung ist es eine Sitzeinrichtung vorzuschlagen, bei der nicht nur die Position des darauf Sitzenden und dessen Bewegungen erfasst wird, sondern die individualisiert werden kann.

### Beschreibung

Die Erfindung betrifft eine Sitzeinrichtung und insbesondere einen Bürosessel. Die Sitzeinrichtung umfasst, wie aus dem Stand der Technik bekannt, ein Sitzelement und eine Rückenlehne. Das Sitzelement und die Rückenlehne sind an einem Trägergestell über eine Vielzahl von Gelenken angeordnet. Das Sitzelement und die Rückenlehne können daher Kippbewegungen und/ oder Linearbewegungen erfahren, wenn sich der Benutzer nach hinten lehnt.

Erfindungsgemäss wird die Aufgabe bei einer Sitzeinrichtung gemäss Oberbegriff des Anspruchs 1 dadurch gelöst, dass die Sensoren von der Sitzeinrichtung erzeugte physikalische und/oder chemische Eigenschaften erfassen. Die Sensoren können nicht nur dazu genutzt werden, die Sitzposition eines Benutzers zu erfassen, wie dies aus dem Stand der Technik bekannt ist, sondern können jede sensorisch erfassbare und während der Benutzung und bei Leerstehen der Sitzeinrichtung erzeugte Eigenschaft der Sitzeinrichtung in ein elektrisches Signal transformieren. Dadurch ist es möglich, die Sitzeinrichtung einer Kostenstelle zuzuordnen und über den Abnutzungsgrad oder den Verschmutzungsgrad der Sitzeinrichtung jederzeit informiert zu sein. Denkbar ist es auch, die Sensorsignale dazu zu benutzen, die Sitzeinrichtung zu individualisieren und zu personalisieren, wie dies von anderen technischen Gebrauchsgegenständen, beispielsweise Smartphones, bekannt ist.

In einer bevorzugten Ausführungsform erfassen die Sensoren eine Benutzung der Sitzeinrichtung, die Art der Benutzung, die Intensität der Benutzung und die damit verbundenen mechanischen Belastungen der Sitzeinrichtung. Aus diesen Messwerten ist der Abnutzungsgrad der Sitzeinrichtung bestimmbar. Dementsprechend können auch Serviceintervalle angezeigt werden. Beispielsweise kann transparent gemacht werden, wann die Gasfeder oder die Gelenke eines Bürosessels ersetzt oder gepflegt werden müssen. Auch ist es denkbar, dass über eine notwendige Reinigung informiert wird. Dies kann insbesondere in medizinischen oder in hygienischen Arbeitsbereichen gefragt sein.

Zweckmässigerweise erfassen die Sensoren die Abnutzung von an der Sitzeinrichtung verbauten Verschleissteilen. Wie im letzten Absatz bereits ausgeführt, können durch dieses Erfindungsmerkmal ein notwendiges Service der Sitzeinrichtung transparent gemacht werden. Auch kann ein notwendiger Pflegebedarf angezeigt werden, wodurch ein teurer Austausch eines Verschleissteiles verhindert werden kann.

In einer weiteren Ausgestaltungsform der Erfindung erkennen die Sensoren einen individuellen Benutzer der Sitzeinrichtung. Die Benutzererkennung kann durch Drucksensoren, welche das Gewicht und die von einem Besitzer eingenommene Sitz- und Lehnenfläche erfassen, umgesetzt sein. Denkbar ist auch ein Fingerabdrucksensor zur Erkennung des Benutzers.

In einer weiteren Ausgestaltungsform umfasst die Sitzeinrichtung einen Prozessor, welcher die Sensordaten erfasst und verarbeitet. Dieses Erfindungsmerkmal ermöglicht es, die Sensordaten mit einer Anwendungssoftware zu visualisieren und weiter zu verarbeiten. Denkbar ist es durch das Vorsehen eines Prozessors, die Sitzeinrichtung mit einem Mess- und Regelsystem auszurüsten und mit unterschiedlicher Anwendungssoftware (sog. "Apps") zu bedienen.

In einer weiteren besonders bevorzugten Ausführungsform ist der Prozessor in ein Computernetzwerk, bevorzugt durch eine Funkverbindung, einbindbar. Zur Netzwerkeinbindung wird die WIFI oder Bluetooth Verbindung bevorzugt angewendet. Dadurch kann in einem Netzwerk die Sitzeinrichtung sichtbar gemacht werden und verschiedene Informationen sind im Netzwerk visualisierbar. Für eine Betriebsverwaltung kann es zum Beispiel hilfreich sein, in einem Computernetzwerk zu erkennen, ob und von wem eine Sitzeinrichtung benutzt wird. Denkbare weitere Nutzungsmöglichkeiten der Netzwerkeinbindung einer Sitzeinrichtung sind eine Reservierung der Sitzeinrichtung oder die Einspielung einer neuen Anwendungssoftware auf den Prozessor. Wird der Sitzeinrichtung eine externe IP Adresse zugeordnet, so kann die erfindungsgemässe Sitzeinrichtung auch von extern, beispielsweise über ein Smartphone mit einer entsprechenden Anwendungssoftware angesprochen werden.

In einer weiteren bevorzugten Ausführungsform der Erfindung ist der Prozessor durch eine Anwendungssoftware bedienbar, die erfassten Daten sind durch die Anwendungssoftware abrufbar und ein Benutzer ist durch die Anwendungssoftware an dem Prozessor anmeldbar. Diese Art der Prozessorbedienung ist Benutzern durch andere Gebrauchsgegenstände geläufig und dementsprechend akzeptiert. Durch eine leicht zu bedienende Benutzeroberfläche lassen sich alle Mess- und Regelaufgaben die erfindungsgemässe Sitzeinrichtung betreffend, einfach lösen. Die Möglichkeit der Anmeldung verstärkt bei dem Benutzer die Wahrnehmung, dass es sich um seine persönliche und von ihm individualisierte Sitzgelegenheit handelt.

In einer weiteren Ausgestaltung der Erfindung ist die Strombedarf der Sensoren und des Prozessors wenigstens teilweise durch die Bewegungen der Sitzeinrichtung generierbar. Um den Einsatz eines Akkumulators zu vermeiden oder dessen Laufzeit zu verlängern, ist es denkbar die zwangsläufig auftretenden Bewegungen der Sitzeinrichtung zu nutzen, um elektrischen Strom zu generieren. Dieses Erfindungsmerkmal kann bei dem Benutzer den positiven Effekt haben, sich verstärkt auf der Sitzeinrichtung zu bewegen. Zu diesen Bewegungen zählen das Rollen, das Hin- und Herdrehen auf der Sitzgelegenheit, das Zurück- und Nach-vor-Kippen der Lehne und das Auf- und Hinsetzen von der Sitzgelegenheit.

Als vorteilhaft erweist es sich, wenn die Sitzeinrichtung durch Stelleinrichtungen, welche mit dem Prozessor in Verbindung stehen, einstellbar ist. Der Prozessor kann demnach nicht nur dazu dienen Messdaten abzurufen, sondern er kann auch dazu dienen, Stelleinrichtungen anzusteuern. Die erfindungsgemässe Sitzeinrichtung lässt sich frei programmieren und genau an die Bedürfnisse eines Benutzers anpassen. Diese Einstellungen lassen sich abspeichern und sind jederzeit wieder abrufbar. So kann eine Sitzeinrichtung, welche von unterschiedlichen Benutzern verwendet wird, rasch auf die jeweiligen abgespeicherten Einstellungen transformiert werden. Die Konfiguration und Einstellung kann durch die Anwendungssoftware erfolgen und kann optional auch manuell direkt an der Sitzgelegenheit erfolgen.

Ein weiterer Aspekt der Erfindung betrifft einen Nachrüstsatz für eine Sitzeinrichtung, welcher die oben beschriebene Vielzahl von Sensoren, einen Prozessor und eine Anwendungssoftware umfasst. Der Nachrüstsatz ermöglicht es, eine Datenerfassung an jeder vorhandenen Sitzgelegenheit nachzurüsten.

Noch ein Aspekt der Erfindung betrifft ein System einer Mehrzahl von erfindungsgemässen Sitzeinrichtungen, welche über eine IP Adresse verfügt und in ein Computer-Netzwerk eingebunden ist. Die dadurch resultierenden Vorteile wurden bereits vorstehend auf Seite 3 ausgeführt.

Zweckmässigerweise ist die Mehrzahl von Sitzeinrichtungen mit einer kabellose Netzwerkverbindung in das Netzwerk eingebunden. Dadurch sind die einzelnen Sitzeinrichtungen in ihren Bewegungsmöglichkeiten durch die Abbindung an das Netzwerk ungestört.

In einer besonders bevorzugten Ausführungsform der Erfindung ist die Mehrzahl von Sitzeinrichtungen mittels einer Anwendungssoftware in dem Computernetzwerk visualisierbar, erfasste Daten jeder Sitzeinrichtung sind abrufbar und jede Sitzeinrichtung ist ansprechbar bzw. ansteuerbar. Dadurch ist jede in das Netzwerk eingebundene Sitzeinrichtung weltweit ansprechbar. Dies ist insbesondere für eine Fernwartung oder eine Femabfrage von Bedeutung.

Aus obigem Absatz folgt, dass eine Sitzeinrichtung des Netzwerkes über das Netzwerk auf die Einstellungen eines Benutzers bevorzugt einstellbar ist. So können beispielsweise Konferenzstühle oder Wartesessel und generell Sitzeinrichtungen, welche von mehreren Benutzern benutzt werden, bereits vorab auf einen Benutzer eingestellt werden.

Weitere Vorteile und Merkmale ergeben sich aus der nachfolgenden Beschreibung von Ausführungsbeispielen der Erfindung.

Die vorliegende Erfindung betrifft eine Sitzeinrichtung und bevorzugt einen Bürosessel. Die Erfindung trägt den gesteigerten Anforderungen Rechnung, Gebrauchsgegenstände verstärkt zu personalisieren und zu individualisieren. Auch berücksichtigt die Erfindung den Trend, dass Firmen die von ihren Mitarbeitern benutzten Büro-Gegenstände wie Bürosessel, Bürotisch oder Computer zentral erfassen und verwalten.

Typischerweise besitzt ein Bürosessel bewegte Teile, beispielsweise eine Gasfeder, welche ein Trägerstell von einem Rollengestell federnd beabstandet. Weitere bewegte Teile eines Bürostuhls sind die Gelenke, welche die Rückenlehne und das Sitzelement des Bürosessels relativ zum Trägergestell beweglich machen. Auch die Rollen des Rollengestells zählen zu den bewegten Teilen. Diese beweglichen Teile sind zwangläufig einer konstanten Abnutzung unterworfen. Auch der Schaumstoff und der Überzug der Rückenlehne und des Sitzelements sind Abnutzungen unterworfen.

Die Bewegungen vorstehend beschriebener Teile lassen sich durch Bewegungssensoren erfassen. Die von den Bewegungssensoren erfassten Daten werden an einen Prozessor weitergeleitet, dort gespeichert und mit in einem Speicher hinterlegten Daten verglichen. Der Einsatz von Sensoren ist bei einem Bürostuhl jedoch nicht auf Bewegungssensoren beschränkt. Denkbar ist auch der Einsatz von Drucksensoren zur Erfassung des Gewichtes und der Bewegungen eines Benutzers oder Fingerabdrucksensoren zur Anmeldung eines Benutzers.

Der mit den Sensoren verbundene Prozessor kann in ein Computernetzwerk eingebunden sein. Dies ist durch die gängigen kabellosen Verbindungstechnologien wie WLAN (WIFI) einfach zu realisieren.

Mit einer an die erfassten Daten angepassten Anwendungssoftware lassen sich die in dem Prozessor abgespeicherten Daten abrufen, analysieren und in Computerprogramme einlesen. So ist es möglich, die Einsatzdauer und den Belastungsgrad (Abhängig von dem Gewicht des Benutzers) der bewegten Teile anzuzeigen und einen notwendigen Austausch gegen Ersatzteile zu erkennen. Die Auswertung der Messdaten kann noch viele andere Resultate liefern. Denkbar ist die Erfassung des effektiven Sitzens, der Einsatz des Bürostuhls pro Zeiteinheit, die Anzeige einer notwendigen Reinigung usw. Auch logistische Daten können so erfasst werden. Beispielsweise können die Erhaltungskosten des Bürostuhls auf die Kostenstelle eines Mitarbeiters gerechnet werden. Als sehr praktisch kann es sich auch erweisen, wenn im Netzwerk ersichtlich ist, ob ein Bürostuhl momentan oder in Zukunft benutzt wird. So ist eine Stuhlreservierung in einem Konferenzraum online möglich oder es ist aus dem Netzwerk ersichtlich, ob der Mitarbeiter an seinem Platz ist oder nicht.

In einer weiteren Ausführungsform kann der Bürosessel auch elektrische Stelleinrichtungen umfassen. Dadurch ist es denkbar, dass der Bürosessel über die Anwendungssoftware einstellbar ist. Denkbar ist es auch, dass ein Bürosessel, welcher von mehreren Benutzern verwendet wird, sich automatisch auf hinterlegte Benutzereinstellungen einstellt. Dies kann durch Anmeldung mit der Anwendungssoftware oder durch einen Fingerabdrucksensor erfolgen.

## Patentansprüche

1. Sitzeinrichtung, insbesondere Bürosessel, mit
- einem Sitzelement,
- einer Rückenlehne,
- einem Trägergestell an welchem das Sitzelement und die Rückenlehne bevorzugt mit einer Vielzahl von Gelenken angeordnet sind und
- an der Sitzeinrichtung angeordnete Sensoren,
**dadurch gekennzeichnet,**
**dass** die Sensoren von der Sitzeinrichtung erzeugte physikalische und/ oder chemische Eigenschaften erfassen.

2. Sitzeinrichtung nach Anspruch 1, **dadurch gekennzeichnet, dass** die Sensoren eine Benutzung der Sitzeinrichtung, die Art der Benutzung, die Intensität der Benutzung und die damit verbundenen mechanischen Belastungen der Sitzeinrichtung erfassen.

3. Sitzeinrichtung nach Anspruch 1 oder 2, **dadurch gekennzeichnet, dass** die Sensoren die Abnutzung von an der Sitzeinrichtung verbauten Verschleissteilen erfassen.

4. Sitzeinrichtung nach einem der vorangehenden Ansprüche, **dadurch gekennzeichnet, dass** die Sensoren einen individuellen Benutzer der Sitzeinrichtung erkennen.

5. Sitzeinrichtung nach einem der vorangehenden Ansprüche, **dadurch gekennzeichnet, dass** die Sitzeinrichtung einen Prozessor umfasst, welcher die Sensordaten erfasst und verarbeitet.

6. Sitzeinrichtung nach Anspruch 5, **dadurch gekennzeichnet, dass** der Prozessor in ein Computernetzwerk, bevorzugt durch eine Funkverbindung, einbindbar ist.

7. Sitzeinrichtung nach einem der Ansprüche 5 bis 6, **dadurch gekennzeichnet, dass** der Prozessor durch eine Anwendungssoftware bedienbar ist, die erfassten Daten durch die Anwendungssoftware abrufbar sind und ein Benutzer durch die Anwendungssoftware an dem Prozessor anmeldbar ist.

8. Sitzeinrichtung nach einem der Ansprüche 5 bis 7, **dadurch gekennzeichnet, dass** die Strombedarf der Sensoren und des Prozessors wenigstens teilweise durch die Bewegungen der Sitzeinrichtung generierbar ist.

9. Sitzeinrichtung nach einem der Ansprüche 5 bis 8, **dadurch gekennzeichnet, dass** die Sitzeinrichtung durch Stelleinrichtungen, welche mit dem Prozessor in Verbindung stehen, einstellbar ist.

10. Sitzeinrichtung nach Anspruch 9, **dadurch gekennzeichnet, dass** die Stelleinrichtungen durch die Anwendungssoftware ansteuerbar sind.

11. Nachrüstsatz für eine Sitzeinrichtung umfassend
- eine Vielzahl von Sensoren,
- einen Prozessor und
- eine Anwendungssoftware
gemäss einem der vorangehenden Ansprüche.

12. System einer Mehrzahl von Sitzeinrichtungen gemäss einem der Ansprüche 1 bis 10,
**dadurch gekennzeichnet,**
**dass** die Mehrzahl von Sitzeinrichtungen über eine IP-Adresse verfügt und in ein Computer-Netzwerk eingebunden ist.

13. System nach Anspruch 22, **dadurch gekennzeichnet, dass** die Mehrzahl von Sitzeinrichtungen mit einer kabellose Netzwerkverbindung in das Netzwerk eingebunden ist.

14. System nach einem der Ansprüche 12 oder 13, **dadurch gekennzeichnet, dass** die Mehrzahl von Sitzeinrichtungen mittels einer Anwendungssoftware in dem Computernetzwerk visualisierbar ist, erfasste Daten jeder Sitzeinrichtung abrufbar sind und jede Sitzeinrichtung ansprechbar bzw. ansteuerbar ist.

15. System nach einem der Ansprüche 12 bis 14, **dadurch gekennzeichnet, dass** eine Sitzeinrichtung des Netzwerkes über das Netzwerk auf die Einstellungen eines Benutzers einstellbar ist.
